# EUROPEAN PATENT APPLICATION

(11) **EP 4 556 049 A1**
(43) Date of publication of application: **21.05.2025**
(21) Application number: 23856374.6
(22) Date of filing: 25.07.2023
(51) Int. Cl.: A61M 25/09, A61B 17/22

(54) **ACOUSTIC ENERGY GUIDEWIRE SYSTEM AND ENERGY GUIDEWIRE**

(30) Priority: 24.08.2022 CN 202211018682
(71) Applicant: Shanghai Microport Rotapace Medtech Co., Ltd., Shanghai 201203 (CN)
(72) Inventor: ZHAO, Ruoheng, Shanghai 201203 (CN); LIU, Peifeng, Shanghai 201203 (CN); YUE, Bin, Shanghai 201203 (CN); CHANG, Zhaohua, Shanghai 201203 (CN)
(74) Representative: Marks & Clerk LLP
(86) International application number: PCT/CN2023/109031
(87) International publication number: WO 2024/041284

(57) **Abstract**

The present application relates to an acoustic energy guidewire system and an energy guidewire (10). The energy guidewire (10) includes a pushing section (11) and a developing section (12). The pushing section (11) includes a first end and a second end opposite to each other. The first end is configured to be connected to a host (20) or a handle (30). The pushing section (11) is made of a metal material. The developing section (12) includes a mandrel (121) and a coating layer (122) connected to the exterior of the mandrel (121). One of the mandrel (121) and the coating layer (122) is connected to the second end and forms an integrated structure with the pushing section (11), and the other one is made of a metal developing material. The elastic modulus of the pushing section (11) is greater than the elastic modulus of the developing section (12).

## Description

### CROSS-REFERENCE TO RELATED APPLICATIONS

The present application claims priority to Chinese patent application No. 202211018682X, titled "ACOUSTIC ENERGY GUIDEWIRE SYSTEM AND ENERGY GUIDEWIRE", filed with the China National Intellectual Property Administration on August 24, 2022, the entire content of which is incorporated herein by reference.

### TECHNICAL FIELD

The present application relates to the technical field of medical devices, and in particular, to an acoustic energy guidewire system and an energy guidewire.

### BACKGROUND

There are many types of chronic total occlusion lesions. The present application specifically introduces chronic total occlusion (CTO) of the coronary artery as an example, which is one of the difficulties in current interventional treatment. Pathological and imaging studies have confirmed that most CTO lesions have forward or reverse collateral circulation, which maintains a certain blood supply to the distal end of the occluded vascular segment. However, even if the collateral circulation is fully established, its function is only equivalent to the blood supply of 90% stenotic blood vessels, and it can only barely maintain the survival of the myocardium at rest and the blood supply to hibernating myocardium. When myocardial oxygen consumption increases, patients will experience symptoms of myocardial ischemia such as angina pectoris and decreased exercise tolerance. Successful opening of the CTO lesions can relieve patients' angina symptoms, improve left ventricular function, stabilize myocardial electrical activity, and thereby enhance patients' tolerance to future coronary events. The CTO lesions account for approximately 30% of all coronary artery lesions. Compared with general complex lesions, the CTO lesions have a low surgical success rate and a high complication rate. How to improve the success rate of interventional treatment of the CTO lesions has been a topic of concern and discussion.

With the improvement of interventional devices, the accumulation of experience and new understanding of CTO lesion theory, the success rate of interventional treatment of the CTO lesions has increased in recent years. The success rate of interventional treatment of the CTO lesions in many large international interventional centers is greater than 90%, but the overall success rate still needs to be further improved. Analyzing the failure factors of interventional treatment of the CTO lesions, the inability of the guidewire to pass (accounting for 63%-92%) ranked first, including failure to penetrate the proximal/distal occluded fiber cap, enter the false lumen, or perforate, followed by failure of the balloon to pass (accounting for 10%), and finally failure to expand the lesion (accounting for 5%). Therefore, the successful opening of the CTO lesions is closely related to the successful selection and application of guidewires. In addition, CTO endovascular interventional treatment generally requires a long operation time and a large amount of contrast agent, and has a large amount of X-ray exposure, which may increase the patient's risk of complications such as contrast-induced nephropathy, radiation-induced skin damage, and cerebral hemorrhage. It also harms the health of the operator who has been engaged in the CTO endovascular interventional treatment for a long time. Therefore, it is necessary to shorten the operation time, and reduce the amount of contrast agent and X-ray exposure through technical improvements.

In the related art, the method of using active energy such as shock waves and ultrasound to assist the passage of the guidewires has gradually matured. The common opening techniques for the CTO lesions include an antegrade guidewire opening technique and a retrograde guidewire opening technique. Generally, since the antegrade guidewire technique is relatively safe, it is the first option for opening the CTO lesions. Due to the long operation time, a large amount of radiation exposure, a large amount of contrast agent used and easy occurrence of the complications, the retrograde guidewire technique is a secondary option when the antegrade guidewire technique fails. However, the current guidewires have thin diameters and hard distal ends in terms of material and structure to pass through the CTO lesions with severe calcification and hard fiber caps, which requires high operation skills. In addition, the current guidewires cannot meet the requirements of interventional treatment of various chronic total occlusion lesions.

### SUMMARY

According to various embodiments of the present application, an acoustic energy guidewire system and an energy guidewire are provided.

The technical solution is as follows: an energy guidewire is provided, including:
a pushing section including a first end and a second end opposite to each other, the first end being configured to be connected to a host or a handle, and the pushing section being made of a metal material; and
a developing section including a mandrel and a coating layer connected to an exterior of the mandrel, one of the mandrel and the coating layer forming an integrated structure with the second end, and the other of the mandrel and the coating layer being made of a metal developing material, and an elastic modulus of the pushing section being greater than an elastic modulus of the developing section.

In an embodiment, the metal developing material includes one or more of gold, platinum, tungsten, iridium, osmium, rhenium, palladium, tantalum, platinum-gold alloy, platinum-tungsten alloy, platinum-iridium alloy, and platinum-nickel alloy.

In an embodiment, the material of the pushing section is selected from nickel-titanium alloy, nickel-titanium-based alloy, iron-based alloy, stainless steel or cobalt-based alloy.

In an embodiment, the mandrel and the pushing section form an integrated structure, the coating layer is made of the metal developing material, and is disposed on the exterior of the mandrel through electroplating or physical vapor deposition.

In an embodiment, the mandrel includes a diameter-varying section and a straight section, two opposite ends of the diameter-varying section are connected to the second end of the pushing section and the straight section, respectively, and an outer diameter of the diameter-varying section decreases in a direction away from the pushing section.

In an embodiment, an angle a formed by an outer wall of the diameter-varying section and a central axis of the diameter-varying section is within a range of 15° to 40°.

In an embodiment, a spiral structure is formed on an outer wall of the coating layer.

In an embodiment, one or more protrusions are arranged on an outer wall of an end of the mandrel away from the pushing section, the protrusions are circumferentially arranged around the end of the mandrel, and the protrusions are sequentially spaced apart in an extension direction of the mandrel.

In an embodiment, a gap between two adjacent protrusions is within a range of 0.8 mm to 1.2 mm.

In an embodiment, the energy guidewire further includes a connecting section, an end of the connecting section is provided with an interface for connecting to the host or the handle, and the other end of the connecting section is connected to the first end of the pushing section.

In an embodiment, a diameter of the connecting section decreases in a direction toward the pushing section.

In an embodiment, an insertion hole is provided on an end face of the connecting section close to the pushing section, a glue injection hole communicating the insertion hole with an outside of the connecting section is provided on an outer wall of the connecting section, and the first end of the pushing section is inserted into the insertion hole and bonded to the connecting section.

In an embodiment, a length of the developing section is within a range of 6 cm to 40 cm, a diameter of the developing section is within a range of 0.2 mm to 0.8 mm, and the diameter of the developing section is less than or equal to a diameter of the pushing section.

An acoustic energy guidewire system is provided, including the energy guidewire of any one of the above embodiments. The acoustic energy guidewire system further includes a host, and the host is connected to the first end.

In an embodiment, the acoustic energy guidewire system further includes a handle disposed between the host and the first end, the host is connected to the handle through a wire, and the handle is connected to the first end.

One or more embodiments of the present application will be described in detail below with reference to drawings. Other features, objects and advantages of the present application will become more apparent from the description, drawings, and claims.

### BRIEF DESCRIPTION OF THE DRAWINGS

The accompanying drawings constituting a part of the present application are used to provide a further understanding of the present application. The illustrative embodiments of the present application and their descriptions are used to explain the present application and do not constitute an improper limitation on the present application.

In order to describe the technical solutions in the embodiments of the present application more clearly, the accompanying drawings required for describing the embodiments will be briefly introduced as follows. Apparently, the accompanying drawings, in the following description, illustrate merely some embodiments of the present application, for a person of ordinary skill in the art, other drawings can also be obtained according to these accompanying drawings without making any creative efforts.
FIG. 1 is a schematic diagram showing a structure of an acoustic energy guidewire system according to embodiments of the present application.
FIG. 2 is a schematic diagram showing a structure of an energy guidewire according to embodiments of the present application.
FIG. 3 is a schematic diagram showing a structure of an energy guidewire according to other embodiments of the present application.
FIG. 4 is a schematic diagram showing a structure of an energy guidewire according to other embodiments of the present application.
FIG. 5 is a schematic diagram showing a structure of an energy guidewire according to other embodiments of the present application.
FIG. 6 is a schematic diagram showing an enlarged structure at A in FIG. 5.
FIG. 7 is a schematic diagram showing a structure of a connecting section and a pushing section of an energy guidewire according to embodiments of the present application.
FIG. 8 is a schematic diagram showing a structure of an energy guidewire when a developing section is arranged concentrically according to embodiments of the present application.
FIG. 9 is a schematic diagram showing a structure of an energy guidewire when a developing section is arranged eccentrically according to embodiments of the present application.

10: energy guidewire; 11: pushing section; 12: developing section; 121: mandrel; 1211: diameter-varying section; 1212: straight section; 1213: protrusion; 1214: gap; 122: coating layer; 1221: spiral structure; 123: through hole; 13: connecting section; 131: interface; 132: insertion hole; 133: glue injection hole; 20: host; 30: handle; 40: wire.

### DETAILED DESCRIPTION OF THE EMBODIMENTS

In order to make the above objectives, features and advantages of the present application more obvious and understandable, specific implementations of the present application are described in detail below with reference to the accompanying drawings. In the following description, many specific details are set forth in order to fully understand the present application. However, the present application can be implemented in many other ways different from those described herein, and those skilled in the art can make similar improvements without departing from the connotation of the present application. Therefore, the present application is not limited by the specific embodiments disclosed below.

As described in the background technology, the related art has the problem that the operation requirements are high and the current guidewires cannot meet the requirements of interventional treatment of various chronic total occlusion lesions. The applicant has found that the reasons for this problem are as follows. Firstly, energy cannot be effectively transmitted along the guidewire. Secondly, severe fractures occur in a welding point region of the guidewire.

Based on the above reasons, the present application provides an acoustic energy guidewire system and an energy guidewire, which have the capability to quickly penetrate various chronic total occlusion lesions, thereby greatly improving the success rate of surgery, reducing surgery time, and further providing a better treatment effect for patients and being beneficial to the health of the operator.

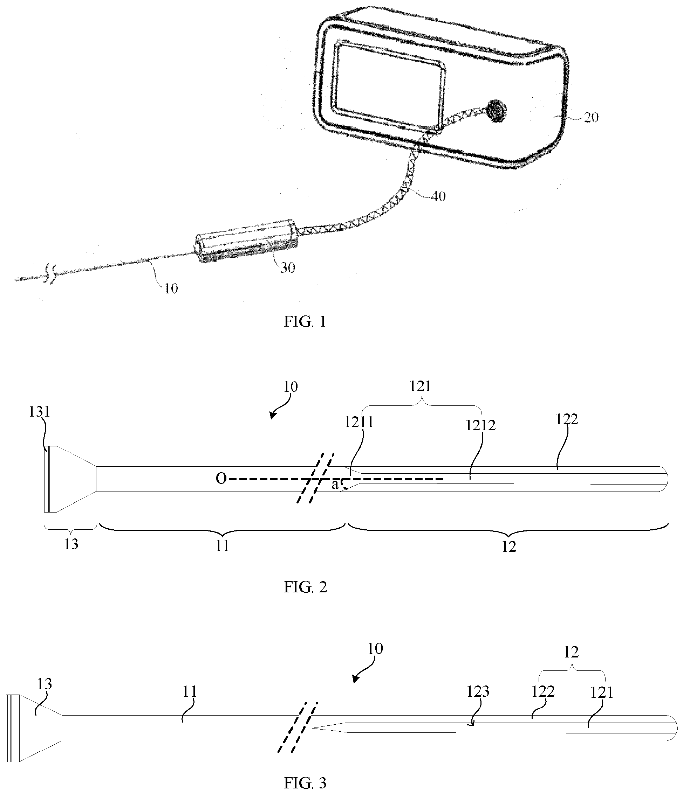

Referring to FIGS. 1 and 2, FIG. 1 is a schematic diagram showing a structure of an acoustic energy guidewire system according to embodiments of the present application, and FIG. 2 is a schematic diagram showing a structure of an energy guidewire according to embodiments of the present application. The embodiments of the present application provide an energy guidewire 10, which includes a pushing section 11 and a developing section 12. The pushing section 11 includes a first end and a second end opposite to each other. The first end is connected to a host 20 or a handle 30. The pushing section 11 is made of a metal material. The developing section 12 includes a mandrel 121 and a coating layer 122 connected to an exterior of the mandrel 121. One of the mandrel 121 and the coating layer 122 is connected to the second end and forms an integrated structure with the pushing section 11, and the other of the mandrel 121 and the coating layer 122 is made of a metal developing material. An elastic modulus of the pushing section 11 is greater than an elastic modulus of the developing section 12.

It should be noted that the integrated structure formed by one of the mandrel 121 and the coating layer 122 and the second end refers to a structure formed by one of the mandrel 121 and the coating layer 122 extending along an axial direction of the pushing section 11 through, for example, the second end, i.e., one of the mandrel 121 and the coating layer 122, and the pushing section 11 are specifically manufactured as a whole using the same metal material.

Optionally, referring to FIG. 2, in the case that the mandrel 121 is connected to the second end and forms the integrated structure with the pushing section 11, when manufacturing the energy guidewire 10, for example, the pushing section 11 and the mandrel 121 are integrally formed by forging, casting, or other processes. For another example, a first semi-finished product including the pushing section 11 can be manufactured first, i.e., the first semi-finished product is manufactured according to a diameter of the pushing section 11, and then a front section of the first semi-finished product is ground to obtain the mandrel 121. Then the coating layer 122 is connected to the exterior of the mandrel 121 through processes including but not limited to electroplating, physical vapor deposition (PVD), etc.

Optionally, referring to FIG. 3, which is a schematic diagram showing a structure of an energy guidewire according to other embodiments of the present application. In the case that the coating layer 122 and the mandrel 121 form an integrated structure, when manufacturing the energy guidewire 10, for example, a second semi-finished product including the pushing section 11 can be manufactured first, i.e., the second semi-finished product is manufactured according to the diameter of the pushing section 11, and then a through hole 123 is formed by drilling from an end face of a front section of the second semi-finished product, and then a metal developing material is injected into the through hole 123 to form the mandrel 121. In this way, the inside of the through hole 123 is filled with the metal developing material, and the material of the coating layer 122 is the same as that of the pushing section 11. Since the coating layer and the pushing section are made of a harder material than the metal developing material, the energy guidewire has better wear resistance and is not easily damaged.

It should be noted that, generally, when an external force is applied to an elastomer, a shape of the elastomer will change (called "deformation"). The general definition of "elastic modulus" is as follows: it is the stress under a uniaxial stress state divided by the strain in the corresponding direction.

When the above energy guidewire 10 is operating, the developing section 12 enters a lesion site under the drive of the pushing section 11 and can apply the energy of the pushing section 11 to the lesion site, thereby playing a therapeutic role. Since one of the mandrel 121 and the coating layer 122 is connected to the second end and forms the integrated structure with the pushing section 11, i.e., a continuous, weld-free, integrated structure, the stress concentration points of the overall mechanical structure of the product are reduced, thereby avoiding rapid failure of the product at the stress concentration point, and ensuring that the energy guidewire 10 can safely and stably transmit mechanical acoustic energy. Furthermore, there is no need to reduce the diameter of the distal part of the energy guidewire 10 (i.e., the part of the energy guidewire 10 away from the handle 30) as utilized in the related art to assist the energy guidewire 10 in entering a tortuous lesion site, thereby avoiding the adverse situation of puncturing blood vessels, i.e., the energy guidewire 10 in this embodiment relatively improves the safety of the operation. Moreover, since the developing section 12 is made of the metal developing material, which has good flexibility, the elastic modulus of the pushing section 11 can be smaller than that of the developing section 12, which can facilitate the developing section 12 to reach the tortuous lesion site, thereby achieving a better treatment effect. In addition, the metal developing material has good X-ray opacity, which facilitates the operator to accurately locate the position of the energy guidewire 10 during the operation. Moreover, since the density of the developable metal material is relatively large, the distal end of the energy guidewire 10 is heavier than that of the energy guidewire of the same volume size in the related art. Therefore, after the same wave source is transmitted to the distal part, the amplitude of the longitudinal wave will decrease, thereby facilitating the energy guidewire 10 to operate stably.

In an embodiment, the metal developing material includes, but is not limited to, one or more of gold, platinum, tungsten, iridium, osmium, rhenium, palladium, tantalum, platinum-gold alloy, platinum-tungsten alloy, platinum-iridium alloy, and platinum-nickel alloy. And/or, optionally, the material of the pushing section 11 includes but is not limited to nickel-titanium alloy, nickel-titanium-based alloy, iron-based alloy, stainless steel or cobalt-based alloy. In this way, based on the material selection of the pushing section 11, it can be achieved that the elastic modulus of the pushing section 11 is greater than the elastic modulus of the developing section 12. The developing section 12 has a relatively low elastic modulus and thus has better flexibility, making it easier to enter into tortuous lesions. Since the elastic modulus of the pushing section 11 is relatively high, it is not easy to bend or knot during the transportation in the catheter, thereby achieving stable transportation of the developing section 12.

It should be noted that the nickel-titanium-based alloy refers to an alloy formed by appropriately reducing the content of Ni element or Ti element in the binary nickel-rich nickel-titanium-based alloy and adding certain Al, Nb, Hf, Ta, Zr, Mo, and Co alloy elements, thereby improving the hardness, high temperature stability, wear resistance, corrosion resistance and other properties while maintaining the various properties of the nickel-rich nickel-titanium-based alloy. Alternatively, certain V, Cu, Hf, Fe, and Cr alloy elements are added to improve the toughness of the nickel-titanium-based alloy, thereby improving the processing performance of the alloy.

It should be noted that iron-based alloy includes but is not limited to iron-nickel alloy, iron-titanium alloy and the like. In addition, the cobalt-based alloy includes but is not limited to cobalt-chromium alloy, cobalt-nickel alloy, and the like.

In a specific embodiment, the mandrel 121 and the pushing section 11 form an integrated structure, and the coating layer 122 is made of the metal developing material and is disposed on the exterior of the mandrel 121 through electroplating or physical vapor deposition. In this way, compared with the method of setting the metal developing material inside the through hole 123, the method of using the metal developing material to make the coating layer 122 not only reduces the production difficulty, but also achieves a larger developing area and good X-ray opacity, facilitating the operator to accurately locate the position of the energy guidewire 10 during the operation.

Referring to any one of FIGS. 2 to 5, in an embodiment, in order to ensure that the pushing section 11 is stably transported inside the catheter to avoid bending and knotting, and to ensure the flexibility of the developing section 12 to enable it to smoothly pass through the tortuous lesion, the diameter of the pushing section 11 is larger than that of the mandrel 121 in the present embodiment.

Furthermore, as an example, the diameter of the pushing section 11 is the same or substantially the same as the diameter of the developing section 12. Being substantially the same means that the diameter of the developing section 12 and the diameter of the pushing section 11 are not absolutely the same in a mathematical sense, but may have a deviation within an allowable range, for example, within 10%. In this way, the developing section 12 can ensure its own flexibility to pass through the tortuous lesion, and simultaneously, its diameter is large enough to avoid the adverse situation of puncturing blood vessels, thereby improving safety performance.

Referring to any one of FIGS. 2 to 5, as an example, the diameter of the pushing section 11 remains constant or substantially constant from the front to the rear. In this way, the pushing section 11 can be conveniently manufactured and formed in one piece. Substantially remaining constant means that the diameter of the pushing section 11 does not remain absolutely constant from the front to the rear in a mathematical sense, but may have a deviation within an allowable range, for example, within 10%. As some optional solutions, the pushing section 11 can also be designed into multiple parts, and the diameters of the parts are not exactly the same. The specific design can be flexibly adjusted and selected according to an actual need, and is not limited here.

Referring to any one of FIGS. 2 to 5, in an embodiment, the pushing section 11 is specifically a solid structure, which can be easily processed and manufactured, and has a relatively high elastic modulus. The pushing section 11 can also be designed as a hollow structure.

Referring to FIG. 2, in an embodiment, the mandrel 121 includes a diameter-varying section 1211 and a straight section 1212. Two opposite ends of the diameter-varying section 1211 are connected to the second end of the pushing section 11 and the straight section 1212, respectively. An outer diameter of the diameter-varying section 1211 decreases in a direction away from the pushing section 11. Specifically, the diameter-varying section 1211 has but is not limited to a cone shape, the diameter of one end of the diameter-varying section 1211 is the same as the diameter of the second end of the pushing section 11, and the diameter of the other end of the diameter-varying section 1211 is the same as the diameter of the straight section 1212. In this way, since the diameter of the second end of the pushing section 11 is larger than that of the straight section 1212, the pushing section 11 transitions to the straight section 1212 through the diameter-varying section 1211 with a decreasing diameter. The design of the diameter-varying section 1211 can effectively facilitate the conduction of acoustic energy while reducing local stress concentration.

Referring to FIG. 2, in an embodiment, an angle formed by an outer wall of the diameter-varying section 1211 and a central axis of the diameter-varying section 1211 (as shown by the dotted line O in FIG. 2) is a. A range of the angle a includes but is not limited to 15° to 40°. Thus, the angle a is designed within this range, which can effectively facilitate the conduction of acoustic energy while reducing local stress concentration.

Specifically, the angle a is, for example, 20°, 22°, 24°, 25°, 27°, or 30°. In the present embodiment, the angle a is 25°.

As some optional solutions, the angle a can also be designed to be any value in the range of 0° to 15°, or 40° to 90°.

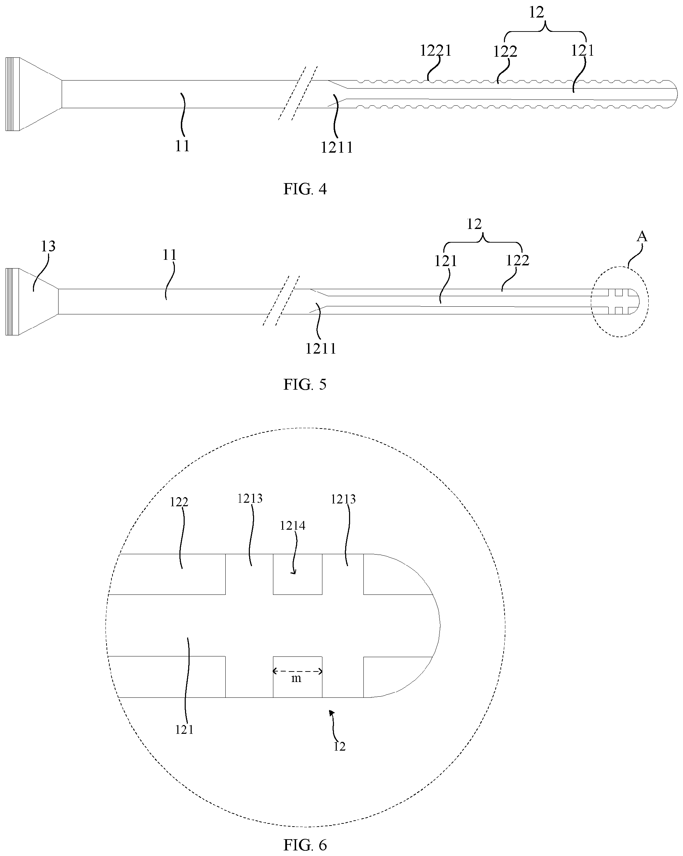

Referring to FIG. 4, FIG. 4 is a schematic diagram showing a structure of an energy guidewire 10 according to other embodiments of the present application. In an embodiment, a spiral structure 1221 is formed on an outer wall of the coating layer 122. In this way, by adding the spiral structure 1221 to destroy the continuous structure of the side surfaces of the coating layer 122, the tensile stress of the curved convex surface during the bending process of the material can be released, and the gaps provide sufficient space to release the compressive stress on the inner surface of the material, thereby further improving the flexibility of the developing section 12. In addition, the spiral structure 1221 can be formed through metal processing using tools such as a lathe. Optionally, the spiral structure 1221 is a spiral groove wound on the outer wall of the coating layer 122.

Referring to FIG. 5 and FIG. 6, FIG. 5 is a schematic diagram showing a structure of an energy guidewire 10 according to other embodiments of the present application, and FIG. 6 is a schematic diagram showing an enlarged structure at A in FIG. 5. In an embodiment, one or more protrusions 1213 are arranged on an outer wall of an end of the mandrel 121 away from the pushing section 11, and the protrusions 1213 are circumferentially arranged around the end of the mandrel 121. A plurality of protrusions 1213 are sequentially spaced apart in an extension direction of the mandrel 121. In this way, each protrusion 1213 is equivalent to an amplification point, and the amplification point has better mechanical transmission. However, since the position of the protrusion 1213 occupies space, the metal developing material cannot be arranged, resulting in a weakened developing effect. In addition, each protrusion 1213 facilitates enhancing the contrast of local development, thereby improving the performance of the energy guidewire 10. Moreover, when a plurality of amplification points are arranged on the outer wall of one end of the developing section 12, a gap 1214 (as shown by arrow m in FIG. 6) is formed between two adjacent protrusions 1213, and the gap 1214 is filled with the metal developing material to improve the developing effect.

Referring to FIG. 6, in an embodiment, the protrusions 1213 and the mandrel 121 form an integrated structure, i.e., the protrusions 1213 and the mandrel 121 are made of the same material and are integrally formed.

It should be noted that, the outer wall of the end of the mandrel 121 is provided with, for example, one, two, three, four or other number of protrusions 1213, and the specific number is not limited here and can be flexibly adjusted and set according to an actual need. When the number of the protrusions 1213 is one, the best mechanical amplification effect is achieved. When the number of the protrusions 1213 is set to be greater than one, this can assist the doctor in measuring the depth of the lesion and speed up the treatment process.

Referring to FIG. 6, in an embodiment, the gap 1214 (as shown by arrow m in FIG. 6) between two adjacent protrusions 1213 is within a range of 0.8 mm to 1.2 mm. Specifically, the gap 1214 between two adjacent protrusions 1213 is set to 0.8 mm, 0.9 mm, 1 mm, 1.1 mm, or 1.2 mm. In this embodiment, the gap 1214 is preferably set to 1 mm, which can assist the doctor in measuring the depth of the lesion and facilitate a more intuitive measurement.

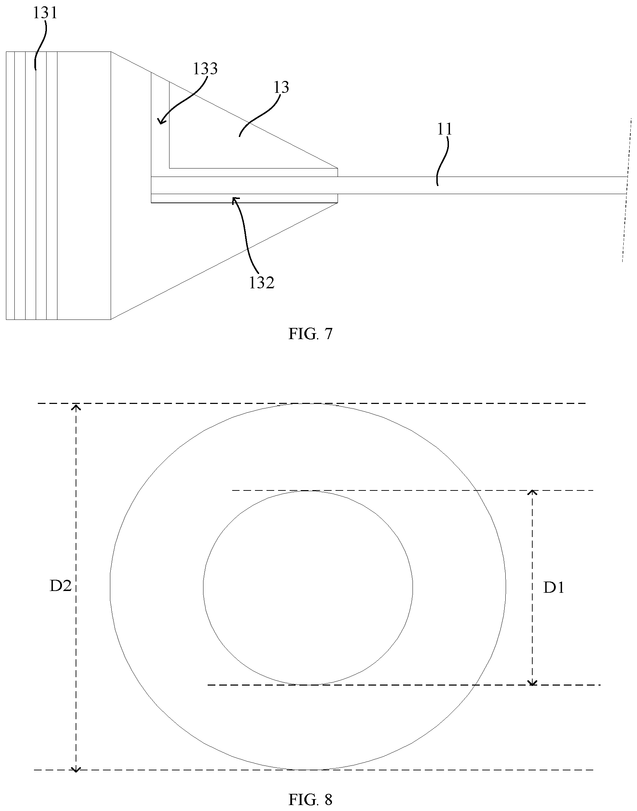

Referring to FIG. 7, FIG. 7 is a schematic diagram showing a structure of a connecting section 13 and a pushing section 11 of an energy guidewire 10 according to embodiments of the present application. In an embodiment, the energy guidewire 10 further includes the connecting section 13. An end of the connecting section 13 is provided with an interface 131 for connecting to the host 20 or the handle 30, and the other end of the connecting section 13 is connected to the first end of the pushing section 11.

Referring to FIG. 7, in an embodiment, a connection method between the interface 131 and the host 20 or the handle 30 includes but is not limited to bonding, mechanical interlocking, threaded assembly, etc. The connection method is not limited here, and can be flexibly adjusted and set according to an actual need.

Referring to FIG. 7, in an embodiment, in order to ensure the transmission effect of acoustic energy, the connecting section 13 is made of metal, preferably the same material as that of the pushing section 11.

Referring to FIG. 7, in an embodiment, a diameter of the connecting section 13 decreases in a direction toward the pushing section 11. And/or, an insertion hole 132 is provided on an end face of the connecting section 13 close to the pushing section 11. A glue injection hole 133 communicating the insertion hole 132 with an outside of the connecting section 13 is provided on an outer wall of the connecting section 13. The first end of the pushing section 11 is inserted into the insertion hole 132 and bonded to the connecting section 13. In this way, when assembling the first end of the pushing section 11 and the connecting section 13, the first end is first inserted into the insertion hole 132, and then glue is injected through the glue injection hole 133, so that the first end and the pushing section 11 can be firmly connected and assembled together.

Referring to FIG. 7, in an embodiment, the connecting section 13 is designed to be assembled with the handle 30 or the host 20 as a part of the handle 30 or the host 20. In other words, the connecting section 13 is designed to be a part of the handle 30 or the host 20, so that the connecting section 13 can remove the interface 131 and directly form an integrated structure with the handle 30 or the host 20.

It should be noted that a length of the pushing section 11 typically constitutes the primary portion of an effective length of the energy guidewire 10, and the developing section 12 is located solely at the distal end of the energy guidewire 10. A length of the developing section 12 is a length that the product may enter into the tortuous lesion, ranging from about 6 cm to 40 cm, preferably 10 cm. Meanwhile, the effective length of the energy guidewire 10 is determined according to an application scenario, and the length of the pushing section 11 is determined by a difference between the above two lengths (the effective length and the length of the developing section 12). The effective length of the energy guidewire 10 is flexibly set and selected according to a usage scenario, which is not limited here. For example, for coronary products, the effective length of the energy guidewire 10 is generally within a range of 1.2m to 1.8m. For another example, for peripheral products, the effective length of the energy guidewire 10 is generally within a range of 1.2m to 3m.

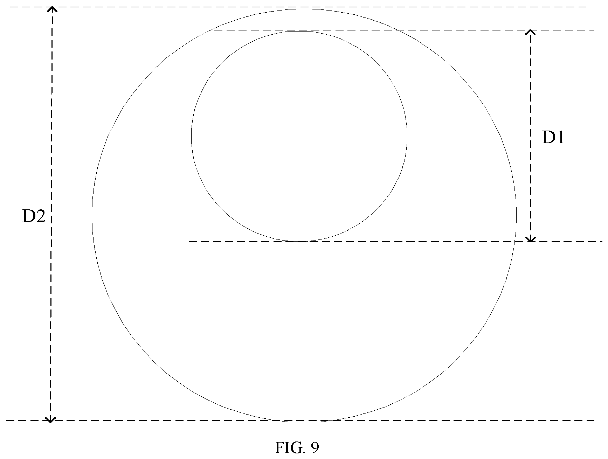

Referring to FIGS. 8 and 9, FIG. 8 is a schematic diagram showing a structure of an energy guidewire 10 when a developing section 12 is arranged concentrically according to embodiments of the present application, and FIG. 9 is a schematic diagram showing a structure of an energy guidewire 10 when a developing section 12 is arranged eccentrically according to embodiments of the present application. In an embodiment, the coating layer 122 and the mandrel 121 can be arranged concentrically, as shown in FIG. 8. The coating layer 122 and the mandrel 121 can also be arranged non-concentrically, i.e., eccentric arrangement, as shown in FIG. 9.

Optionally, referring to FIG. 9, when the coating layer 122 and the straight section 1212 of the mandrel 121 are arranged eccentrically, the mandrel 121 is not arranged at a center of the energy guidewire 10. Due to the significant difference in density between the two materials, a center of gravity of the entire product is offset to a certain extent from a geometric center of the outer contour. This solution can reduce the interference of longitudinal waves on the operation of the energy guidewire 10. During the transmission of the longitudinal waves, the geometric center of the energy guidewire 10 in a vibration direction of the wave is not the center of gravity, so additional inelastic energy dissipation is generated during the sinusoidal oscillation, thereby reducing the overall impact of the longitudinal vibration.

After extensive research, it was found that for the energy guidewire 10 in which the mandrel 121 and the pushing section 11 form an integrated structure, when a ratio of a diameter D1 of the mandrel 121 to a diameter D2 of the developing section 12 changes, a bending strength of the developing section 12 changes accordingly. In order to maintain the bending strength of the energy guidewire 10 with developing sections 12 of different specifications at an adjustable level, the bending strengths under different application requirements can be calculated based on the bending stiffness table and experimental results.

The following is a table showing the bending stiffness, and the table shows a relationship between the bending stiffness and the diameter ratio of two energy guidewires 10 of different specifications:

### Bending stiffness table

| Guidewire Specification | 0.014" | 0.018" |
|---|---|---|
| Bending stiffness | The ratio of the diameter of mandrel 121 to the diameter of developing section 12 (D1/D2) | |
| High | (35%, 60%) | (27%, 45%) |
| Middle | (24%, 35%) | (18%, 27%) |
| Low | (12%, 24%) | (7%, 18%) |

| | | |
|---|---|---|
| Note: the values of the energy guidewire 10 in the table correspond to the following material combination: the mandrel 121 is made of a nickel-titanium alloy (1:1), and the coating layer 122 is made of pure gold. The mandrel 121 and the coating layer 122 are configured in a concentric structure shown in FIG. 8. In addition, the percentage in the table is the ratio of the diameter of the mandrel 121 to the diameter of the developing section 12, i.e., D1/D2. The non-concentric structure is illustrated in FIG. 9. D1 and D2 represent the same meaning, but the specific outer diameter ratio is different. For the non-concentric design, a design with a specified lower limit percentage (a smaller percentage) can be used. | | |

The calculation formula for the bending stiffness is denoted as: K=EI, where K represents the bending stiffness (unit: Pa*m^4), indicating the extent to which a component can be easily bent, E (unit: Pa) represents the bending elastic modulus of the material (metals are typically polycrystalline and therefore not anisotropic, and the bending elastic modulus can be calculated using the elastic modulus of normal stress), and I represents the moment of inertia of the section (unit: m^4), which is determined by the cross-sectional shape of the component, such as variations in the outer diameter of the guidewire product.

The levels of the bending stiffness in the current design are graded as follows:
High level: not suitable for tortuous lesions and coronary arteries, suitable for the above-knee segments of peripheral blood vessels, with strong pushing performance and the highest efficiency in acoustic wave transmission;
Middle level: suitable for non-tortuous diseased segments of coronary arteries or below-the-popliteal segments of peripheral blood vessels; and
Low level: suitable for lesions of conventional coronary arteries or lesions with moderate tortuosity in the coronary arteries.

Referring to FIG. 1 and FIG. 2, in an embodiment, an acoustic energy guidewire system is provided, including the energy guidewire 10 of any one of the above embodiments. The acoustic energy guidewire system further includes a host 20 connected to the first end. The energy guidewire 10 is configured to transmit acoustic wave energy along a treatment path for treatment. The host 20 provides energy for the entire acoustic energy guidewire system. In addition, optionally, the host 20 further provides a human-computer interaction interface and treatment parameter settings.

When the above acoustic energy guidewire system is operating, the developing section 12 enters a lesion site under the drive of the pushing section 11 and can apply the energy of the pushing section 11 to the lesion site, thereby playing a therapeutic role. Since one of the mandrel 121 and the coating layer 122 is connected to the second end and forms the integrated structure with the pushing section 11, i.e., a continuous, weld-free, integrated structure, the stress concentration points of the overall mechanical structure of the product are reduced, thereby avoiding rapid failure of the product at the stress concentration points, and ensuring that the energy guidewire 10 can safely and stably transmit mechanical acoustic energy. Furthermore, there is no need to reduce the diameter of the distal part of the energy guidewire 10 (i.e., the part of the energy guidewire 10 away from the handle 30) as utilized in the related art to assist the energy guidewire 10 in entering a tortuous lesion site, thereby avoiding the adverse situation of puncturing blood vessels, i.e., the energy guidewire 10 in this embodiment relatively improves the safety of the operation. Moreover, since the developing section 12 is made of the metal developing material, which has good flexibility, the elastic modulus of the pushing section 11 can be smaller than that of the developing section 12, which can facilitate the developing section 12 to reach the tortuous lesion site, thereby achieving a better treatment effect. In addition, the metal developing material has good X-ray opacity, which facilitates the operator to accurately locate the position of the energy guidewire 10 during the operation. Moreover, since the density of the developable metal material is relatively large, the distal end of the energy guidewire 10 is heavier than that of the energy guidewire of the same volume size in the related art. Therefore, after the same wave source is transmitted to the distal part, the amplitude of the longitudinal wave will decrease, thereby facilitating the energy guidewire 10 to operate stably.

In an embodiment, the acoustic energy guidewire system further includes a handle 30 disposed between the host 20 and the first end. The host 20 is connected to the handle 30 through a wire 40, and the handle 30 is connected to the first end. The handle 30 can improve the operability of the entire device. The wire 40 is configured to connect an energy and signal path between the handle 30 and the host 20, so that the communication between the handle 30 and the host 20 is not limited by the distance, thereby greatly improving the operability.

In some embodiments, the handle 30 and the wire 40 may also be removed. In other words, optionally, the host 20 is directly connected to the energy guidewire 10. Accordingly, the effective length of the energy guidewire 10 can be increased to ensure operability. In addition, the operation control functions originally on the handle 30 are transferred to the host 20 and implemented through the host 20.

In some embodiments, an energy conversion component is provided inside the acoustic energy guidewire system. Common principles include: hydraulic electric type, piezoelectric type, electromagnetic type, holmium laser type, pneumatic impact type, etc. The energy conversion component is typically located inside the handle 30 or the host 20. When the acoustic energy guidewire system is provided with the handle 30, a transducer is located inside the handle 30. When the handle 30 is removed from the acoustic energy guidewire system, the transducer is mounted inside the host 20. Regardless of the design, the energy guidewire 10 is directly (possibly through a rigid connection such as an amplifier rod, or a lock) connected to an acoustic energy output portion of the transducer.

The technical features in the above embodiments may be combined arbitrarily. For concise description, not all possible combinations of the technical features in the above embodiments are described. However, provided that they do not conflict with each other, all combinations of the technical features are to be considered to be within the scope described in this specification.

The above-mentioned embodiments only describe several implementations of the present application, and their description is specific and detailed, but should not be understood as a limitation on the patent scope of the present application. It should be noted that, for a person of ordinary skill in the art may further make variations and improvements without departing from the conception of the present application, and these all fall within the protection scope of the present application. Therefore, the protection scope of the present application should be subject to the appended claims.

In addition, the terms such as "first" and "second" are used for descriptive purposes only, and should not be understood as indicating or implying relative importance or implicitly indicating the quantity of the technical features indicated. Thus, the features described with "first" and "second", etc., may explicitly or implicitly include at least one of these features. In the description of the present application, the term "plurality" means at least two, such as two, three, etc., unless otherwise clearly and specifically defined.

In the present application, unless otherwise clearly specified and limited, the terms "mounted", "coupling", "connection", "fixation", etc., should be understood in a broad sense, for example, it may be a fixed connection, a detachable connection, or integration. It may be a mechanical connection or an electrical connection. It may be a direct connection or an indirect connection through an intermediate medium. It may be an internal connection between two elements or an interaction relationship between the two elements, unless otherwise clearly defined. Those of ordinary skill in the art can understand the specific meanings of the above terms in the present application according to specific situations.

It should be noted that when an element is referred to as being "fixed on" or "disposed on" another element, it may be directly on the other element or there may be an intervening element. When an element is referred to as being "connected to" another element, it may be directly connected to the other element or there may also be an intermedium element. The terms "vertical", "horizontal", "upper", "lower", "left", "right" and similar expressions used in this application are for the purpose of illustration only and are not meant to be the only implementation methods.

## Claims

1. An energy guidewire, comprising:
a pushing section comprising a first end and a second end opposite to each other, the first end being configured to be connected to a host or a handle, and the pushing section being made of a metal material; and
a developing section comprising a mandrel and a coating layer connected to an exterior of the mandrel, one of the mandrel and the coating layer forming an integrated structure with the second end, and the other of the mandrel and the coating layer being made of a metal developing material, and an elastic modulus of the pushing section being greater than an elastic modulus of the developing section.

2. The energy guidewire according to claim 1, wherein the metal developing material comprises one or more of gold, platinum, tungsten, iridium, osmium, rhenium, palladium, tantalum, platinum-gold alloy, platinum-tungsten alloy, platinum-iridium alloy, and platinum-nickel alloy.

3. The energy guidewire according to claim 1 or 2, wherein the material of the pushing section is selected from nickel-titanium alloy, nickel-titanium-based alloy, iron-based alloy, stainless steel or cobalt-based alloy.

4. The energy guidewire according to any one of claims 1 to 3, wherein the mandrel and the pushing section form an integrated structure, the coating layer is made of the metal developing material, and is disposed on the exterior of the mandrel through electroplating or physical vapor deposition.

5. The energy guidewire according to claim 4, wherein the mandrel comprises a diameter-varying section and a straight section, two opposite ends of the diameter-varying section are connected to the second end of the pushing section and the straight section, respectively, and an outer diameter of the diameter-varying section decreases in a direction away from the pushing section.

6. The energy guidewire according to claim 5, wherein an angle a formed by an outer wall of the diameter-varying section and a central axis of the diameter-varying section is within a range of 15° to 40°.

7. The energy guidewire according to any one of claims 4 to 6, wherein a spiral structure is formed on an outer wall of the coating layer.

8. The energy guidewire according to any one of claims 4 to 7, wherein one or more protrusions are arranged on an outer wall of an end of the mandrel away from the pushing section, the protrusions are circumferentially arranged around the end of the mandrel, and the protrusions are sequentially spaced apart in an extension direction of the mandrel.

9. The energy guidewire according to claim 8, wherein a gap between two adjacent protrusions is within a range of 0.8 mm to 1.2 mm.

10. The energy guidewire according to any one of claims 1 to 9, wherein the energy guidewire further comprises a connecting section, an end of the connecting section is provided with an interface for connecting to the host or the handle, and the other end of the connecting section is connected to the first end of the pushing section.

11. The energy guidewire according to claim 10, wherein a diameter of the connecting section decreases in a direction toward the pushing section.

12. The energy guidewire according to claim 10 or 11, wherein an insertion hole is provided on an end face of the connecting section close to the pushing section, a glue injection hole communicating the insertion hole with an outside of the connecting section is provided on an outer wall of the connecting section, and the first end of the pushing section is inserted into the insertion hole and bonded to the connecting section.

13. The energy guidewire according to any one of claims 1 to 12, wherein a length of the developing section is within a range of 6 cm to 40 cm, a diameter of the developing section is within a range of 0.2 mm to 0.8 mm, and the diameter of the developing section is less than or equal to a diameter of the pushing section.

14. An acoustic energy guidewire system, comprising an energy guidewire according to any one of claims 1 to 13, wherein the acoustic energy guidewire system further comprises a host, and the host is connected to the first end.

15. The acoustic energy guidewire system according to claim 14, wherein the acoustic energy guidewire system further comprises a handle disposed between the host and the first end, the host is connected to the handle through a wire, and the handle is connected to the first end.
